**Europäisches Patentamt**

(19) **European Patent Office**

**Office européen des brevets**

(11) Veröffentlichungsnummer: **0 037 056**
**B1**

(12) # EUROPÄISCHE PATENTSCHRIFT

(45) Veröffentlichungstag der Patentschrift:
08.06.83

(51) Int. Cl.³: **C 12 Q 1/00,** C 12 Q 1/28,
**G 01 N 33/52,** G 01 N 31/22

(21) Anmeldenummer: 81102200.3

(22) Anmeldetag: 24.03.81

(54) **Verfahren und diagnostische Mittel zum Nachweis von Redox-Reaktionen.**

(30) Priorität: 29.03.80 DE 3012368

(43) Veröffentlichungstag der Anmeldung:
07.10.81 Patentblatt 81/40

(45) Bekanntmachung des Hinweises auf die Patenterteilung:
08.06.83 Patentblatt 83/23

(84) Benannte Vertragsstaaten:
AT BE CH DE FR GB IT LI LU NL SE

(56) Entgegenhaltungen:
FR-A-2 339 172
US-H-978 003

CHEMICAL ABSTRACTS, Band 85, Nr. 22, 29. November 1976, Seite 797, Zusammenfassung 171295j, COLUMBUS, OHIO (US). K.N. MURTY et al.: "Determination of ascorbic acid with potassium iodate, iodine, potassium bromate and iodine monochloride using naphtol blue black, amaranth and Brilliant Ponceau 5R as indicators"

CHEMICAL ABSTRACTS, Band 87, Nr. 25, 19. Dezember 1977, Seite 768, Zusammenfassung 201963m, COLUMBUS, OHIO (US). D.N. SAMIOS et al.: "Investigation on the rate law of the reaction of ascorbic and iodate. Calculation of kinetic parameters applying stopped-flow techniques"

(73) Patentinhaber: BOEHRINGER MANNHEIM GMBH,
Sandhofer Strasse 112-132 Postfach 31 01 20,
D-6800 Mannheim 31-Waldhof (DE)

(72) Erfinder: Koevér, Laszlo, Dipl.Ing., Lampertheimer Strasse 107/A, D-6800 Mannheim 31 (DE)
Erfinder: Rittersdorf, Walter, Dr.rer.nat., Kasseler Strasse 6, D-6800 Mannheim 31 (DE)
Erfinder: Werner, Wolfgang, Dr.rer.nat., Meissener Weg 39, D-6800 Mannheim-Vogelstang (DE)

(56) Entgegenhaltungen:
RESEARCH DISCLOSURE, Band 171, Nr. 17144, Juli 1978, Seiten 41–43, "Reduction of detectable species migration in elements for the analysis of liquids"

## Verfahren und diagnostische Mittel zum Nachweis von Redox-Reaktionen

Die Erfindung betrifft Verfahren und diagnostische Mittel, die auf Redox-Reaktionen beruhen, die Jodate zur Vermeidung von Störungen durch Ascorbinsäure enthalten.

In der klinischen und pharmazeutischen Chemie, der Biochemie und Lebensmittelchemie sind für Bestimmungsmethoden von Substraten und Enzymen Redox-Systeme von grosser Bedeutung. Für solche Bestimmungsmethoden existieren die verschiedensten photometrischen Verfahren. Von besonderer Bedeutung sind jedoch sogenannte Schnelldiagnostica. Das sind Mittel, bei denen alle Reagenzien in saugfähigen Trägern oder in Filmen in trockener Form vorliegen. Die Mittel werden mit der Untersuchungsflüssigkeit in Berührung gebracht, die entstehende Farbe kann visuell oder mit Reflexionsphotometern beurteilt werden.

Die zu untersuchenden Materialien auf den obengenannten Gebieten der chemischen Analytik, wie z.B. Urin, Blut, Lebensmittel, Arzneimittelzubereitungen u.a. sind vielfach gekennzeichnet durch einen mehr oder weniger grossen Gehalt an Reduktionsmitteln, von denen das häufigste die Ascorbinsäure ist. Es ist klar, dass Redox-Reaktionen durch starke Reduktionsmittel wie Ascorbinsäure empfindlich gestört werden können. So ist bekannt, dass beim Nachweis von Glucose mit Schnelldiagnostica, die auf der Basis der Reaktion GOD-POD-Redox-Indikator beruhen, durch Ascorbinsäure falsch negative Befunde hervorgerufen werden. Das aus Glucose mit Hilfe von GOD (Glucoseoxidase) entstandene $H_2O_2$ wirkt nämlich mit POD (Peroxidase) auf die Ascorbinsäure anstatt auf den Indiaktor unter Oxidation ein und wird so der Bestimmung entzogen.

Weiter ist bekannt, dass Schnelldiagnostica zum Nachweis von Blut im Harn ebenfalls in Anwesenheit von Ascorbinsäure falsch negativ reagieren. Vermutlich reduziert hier die Ascorbinsäure den durch die hämoglobinkatalysierte Oxidation gebildeten Farbstoff.

Als Beispiel für falsch positive Befunde durch Ascorbinsäure soll die Bestimmung von NADH oder NADPH mit Hilfe der Reduktion von Tetrazoliumsalzen zu farbigen Formazanen angeführt werden. Ascorbinsäure wirkt hier gleichsinnig und erhöht das Messignal.

Wegen der besonderen Bedeutung und des Umfanges der Störungen durch Reduktionsmittel, insbesondere Ascorbinsäure, hat es daher nicht an Versuchen gefehlt, diese aus den Untersuchungsflüssigkeiten zu entfernen, bzw. nicht gestörte Verfahren und Mittel zu entwickeln.

Folgende Verfahren sind bekanntgeworden:

- Oxidation mit Jodlösung und Entfernung des überschüssigen Jods mit Thiosulfat
- Oxidation mit Mangandioxid und Abfiltrieren des nicht verbrauchten Oxidationsmittels
- Oxidation mit alkalischem $H_2O_2$
- Behandlung der Probelösung mit Anionenaustauscher

Alle diese Verfahren erfordern eine umständliche Behandlung der Probenlösung. des weiteren ist insbesondere bei Schnelldiagnostica eine integrierte Problemlösung sehr aufwendig. Es sind auch Testpapiere bekannt, bei denen Urin zuerst durch eine Zone mit Anionenaustauscher (DE-AS 15 98 008) chromatographieren muss, um dann bei Weiterlaufen auf die eigentliche Reagenzzone ungestört reagieren zu können. Teste mit Ionenaustauscherbezirken sind für Glucose und Galactose handelsüblich. Sie weisen einen komplizierten Aufbau auf und durch die erforderliche Chromatographierzeit wird die Analysenzeit im Vergleich zu herkömmlichen Schnelltesten erheblich gesteigert.

Eine weitere Möglichketi zur Entfernung von Ascorbinsäure aus Flüssigkeiten, bzw. der Entstörung von diagnostischen Mitteln, beruht auf dem Zusatz von Ascorbatoxidase in optischen Testen und Schnelldiagnostica. (DE-AS 26 25 834)

Obwohl dieses Verfahren besonders bei kleinen Ascorbinsäure-Konzentrationen brauchbar ist, kann das Problem als Ganzes nicht als gelöst betrachtet werden, und zwar aus folgenden Gründen:

- Ascorbatoxidase reagiert nur mit Ascorbinsäure selbst, nicht jedoch mit Metaboliten wie dem Glucuronid und dem Sulfat, sowie anderen Reduktionsmitteln.
- Die Oxidation von Ascorbinsäure durch Ascorbatoxidase ist relativ langsam, so dass bei Testflüssigkeiten, die mehr als 100 mg Ascorbinsäure/dl enthalten können, unwirtschaftlich grosse Mengen Ascorbatoxidase eingesetzt werden müssen.
- In bestimmten Fällen wird das Enzym Ascorbatoxidase durch aggressive Reagenzien relativ schnell zerstört.
  So muss z.B. das in einem Harn-Blut-Test verwendete Cumolhydroperoxid in Mikrokapseln eingeschlossen werden (USP 4 129 417).

Es wurde nun überraschend gefunden, dass man Verfahren und diagnostische Mittel, insbesondere Schnelldiagnostica entwickeln kann, die nicht durch Ascorbinsäure und ihre Metaboliten gestört sind, auch wenn diese in relativ grossen Mengen vorhanden sind, wenn man den bekannten Rezepturen oder dem zu untersuchenden Testsystem zusätzlich Jodat zufügt. Es muss als überraschend angesehen werden, dass Jodat zwar die Ascorbinsäure hinreichend schnell oxidiert, nicht jedoch die in der klinischen Chemie wichtigen Substrate, ferner viele der in der Analytik gebräuchlichen Redox-Indikatoren und ihre jeweiligen farbigen Reaktionsprodukte sowie übliche Hilfsstoffe. So werden beispielsweise unter den üblichen Analysenbedingungen (pH 5–9) und innerhalb der üblichen Analysenzeiten von Jodat nicht angegriffen:

– Substrate:
Kohlehydrate (Glucose, Galactose u.a.), Cholesterin, Glycerin (aus Triglyceriden), Harnsäure, NADH und NADHP etc.
– Substratoxidasen:
Glucoseoxidase, Galactoseoxidase, Cholesterinoxidase, Glycerinoxidase, Uricase etc.
– Indikatoren:
Benzidinabkömmlinge (o-Tolidin, 3,3′,5,5′-Tetramethylbenzidin), heterocyclische Azine (Azinobis-benzo-thiazolon-sulfonsäure), Formazane als Reduktionsprodukte von Tetrazoliumsalzen etc.
– Peroxidasen:
Meerrettichperoxidase, Hämoglobin (Blut)
– Hilfsstoffe:
Arylsemicarbazide, die als Stabilisatoren von Oxidationsindikatoren beschrieben sind (DE-OS 27 16 060)

Dass diese Eigenschaft des Jodats überraschend ist, und nicht etwa aus dem Oxidatonspotential abgeleitet werden kann, zeigt ein Vergleich mit anderen Halogen-Verbindungen. Deren Standardpotentiale betragen laut Cotton-Wilkinson «Anorganische Chemie» Weinheim 1970, 2. Auflage, S. 532:

– Jodat + 0,26 V
– Perjodat + 0,39 V
– Jod + 0,54 V
– Bromat + 0,61 V
– Chlorat + 0,63 V

Während nun Perjodat und freies Jod neben Ascorbinsäure die meisten Indikatoren, vor allem die Benzidinabkömmlinge oxidieren, sind Bromat und Chlorat trotz ihrer höheren Oxidationspotentiale nicht in der Lage, Ascorbinsäure zu oxidieren und sind somit für den angestrebten Zweck völlig unbrauchbar. Auch von Jodaten wurde bisher angenommen, dass sie Ascorbinsäure nur in essigsaurer Lösung oxidieren [R. Indovina, D. Elia, Boll. Soc. Ital. Biol. sperm. 20, 390–393 (1945), C.A. 40, 6110 (1946)].

Die erfindungsgemässen, durch Ascorbinsäure praktisch nicht gestörten Schnelldiagnostica sind einfach herzustellen, indem man den Rezepturen an sich bekannter Teste ein Jodat zumischt. Solche Teste sind z.B.:

– Testpapiere zum Nachweis von Blut im Harn mit organischen Hydroperoxiden und o-Tolidin (DE-OS 22 35 152, DE-OS 26 40 211, DE-OS 12 42 905) und Tetramethylbenzidin (DE-OS 24 60 903, DE-OS 27 16 060)
– Testpapiere zum Nachweis von Glucose im Harn mit GOD, POD und o-Tolidin (DE-OS 24 15 257, DE-AS 11 21 847, OE-PS 19 88 96), 3,3′,5,5′-Tetramethylbenzidin (DE-OS 24 60 903), substituierten Aminocarbazolen (DE-OS 22 05 733, DE-OS 23 38 932) und heterocyclischen Azinen (DE-OS 16 48 840)

– Testpapiere zum Nachweis von Galactose im Harn mit Galactose-OD, POD und o-Tolidin (USP 3,362,886)
– Testpapiere diverser Substrate mit spezifischen Oxidasen, POD und o-Tolidin (USP 3,099,605)
– Testfilme zur Bestimmung von Glucose im Blut mit GOD, POD, o-Tolidin (DE-OS 15 98 153) und 3,3′,5,5′-Tetramethylbenzidin (DE-OS 24 60 903)
– Testpapiere zur Bestimmung von NADH oder NADH-bildenden Substraten bzw. Enzymen mit Tetrazoliumsalzen und Diaphorase (z.B. DE-OS 24 52 283)

Da die meisten der eben genannten Teste in wässrigen Lösungen durchgeführt werden, werden vorteilhaft wasserlösliche Jodate verwendet. Es kommen praktisch alle wasserlöslichen Salze der Jodsäure mit anorganischen und organischen Kationen in Frage, soweit diese ihrerseits die analytischen Verfahren nicht stören. Es sind dies vor allem die Alkalisalze, die bequem zugänglich und zum Teil handelsüblich sind, ferner die Erdalkalisalze, Ammoniumsalze oder die Salze einfacher Amine wie z.B. Piperidin, Piperazin u.a.

Nur in besonderen Fällen sind gewisse Modifikationen der üblichen Rezepturen erforderlich:

– Bei der industriellen Herstellung von Testpieren sind mitunter relativ lange Imprägnierzeiten erforderlich. In diesen langen Zeiten kann es unter Umständen in der Imprägnierlösung schon zu einer teilweisen Oxidation des Indikators kommen. Dies ist beispielsweise der Fall bei substituierten Aminocarbazolen gemäss DE-OS 22 05 733. In diesen Fällen ist es zweckmässig, eine gewisse räumliche Trennung der Reagenzien herbeizuführen, indem man zunächst das Testpapier mit allen anderen Reagenzien imprägniert und danach ein entsprechendes Jodat aus einem organischen Lösungsmittel, das die übrigen Rezepturbestandteile nicht auflöst, nachimprägniert. Organolösliche Jodate sind beispielsweise quarternäre Ammoniumjodate, sowie Salze der Jodsäure mit längerkettigen Aminen.
– In Fällen, wo durch die Verwendung von Jodat Stabilitätsprobleme der Teste auftreten, können allgemein bekannte Massnahmen der Stabilitätsverbesserung, wie aufeinanderfolgende Imprägnation aus verschiedenen Lösungsmitteln gegebenenfalls unter Zugabe von geeigneten Trennmitteln wie z.B. Polymeren ergriffen werden.

Die Verwendung von Jodat in Schnelldiagnostica ist nur innerhalb folgender pH-Grenzen zweckmässig:

– Bei pH-Werten unterhalb ca. 4,5 entsteht bei der Reduktion von Jodat durch Ascorbinsäure zunehmend freies Jod, welches wie schon gesagt, viele Indikatoren oxidiert. Ausserdem besitzt Jodat im sauren Milieu ein Oxidationspotential von +1,20 V (Cotton-Wilkinson, siehe oben) und ist daher mit den meisten Indikatoren und anderen Rezepturbestandteilen nicht mehr verträglich.
- Oberhalb von pH 7-8 wird die Oxidation von Ascorbinsäure durch Jodat zunehmend träger, so dass für eine wirksame Entstörung unverhältnismässig grosse Mengen Jodat erforderlich sind, was zu Verarbeitungsschwierigkeiten und gegebenenfalls zu Haltbarkeitsproblemen führen kann.

Die Jodate werden zweckmässigerweise im 2- bis 20fachen molaren Überschuss bezogen auf die in den Untersuchungsflüssigkeiten vorkommenden Mengen an Ascorbinsäure eingesetzt. Da die Oxidation der Ascorbinsäure praktisch vor der eigentlichen Nachweisreaktion ablaufen muss, benötigt man für schnell ablaufende Nachweisreaktionen einen grösseren Überschuss an Jodat als für langsamer ablaufende. Da wie oben schon erwähnt die Oxidation der Ascorbinsäure sich bei hohem pH verlangsamt, muss auch in diesen Fällen ein grösserer Überschuss verwendet werden. Die richtige Menge Jodat ist in jedem Fall durch einfache Reihenversuche leicht zu ermitteln.

Die erfindungsgemässen Schnelldiagnostica weisen gegenüber bekannten störungsfreien bzw. störungsarmen Schnelldiagnostica folgende Vorteile auf:
- Ihre Handhabung entspricht vollkommen der der bisherigen, z.T. seit langem üblichen Schnelldiagnostica.
- Ihre Herstellung ist einfach und entspricht weitgehend üblichen Fabrikationsmethoden.
- Verglichen mit Schnelldiagnostica, die Ascorbatoxidase enthalten, weisen sie z.T. einen höheren Entstörungsgrad auf, vor allem sind sie wesentlich preisgünstiger herzustellen.

Die Anwendung von Jodat ist an sich nicht auf die Incorporierung in Schnelldiagnostica beschränkt. So kann z.B. Jodat direkt der zu untersuchenden Lösung zugesetzt werden und die dadurch von den störenden Reaktionsmitteln befreite Lösung in bekannter Weise photometrisch oder mit üblichen Schnelltesten weiter untersucht werden. Es sei allerdings angemerkt, dass in einer solchen Lösung Teste, deren Substrate oder Reagenzien mit Jodat reagieren, nicht mehr durchgeführt werden können.

Es sind beispielsweise bei der Harnuntersuchung mit Mehrfach-Testen die Teste auf Nitrit und Gallenfarbstoffe (Urobilinogen und Bilirubin). Diese Stoffe werden von Jodat im sauren Milieu des Testpapieres noch vor ihrem Nachweis oxidiert. Ausserdem werden die für die üblichen Nitrit-Teste verwendeten aromatischen Amine zu stark farbigen Verbindungen oxidiert.

Ob ein bestimmter Test durch den Zusatz von Jodat gestört wird, lässt sich einfach nachprüfen, indem man Standards mit und ohne Jodatzusatz vergleicht.

In gewissen Fällen, z.B. bei Analysenverfahren, die bei pH 5-6 arbeiten, wo Ascorbinsäure sehr schnell von Jodat oxidiert wird, kann es vorteilhaft sein, das Jodat der Reagenzienzubereitung oder Teilen derselben zuzugeben und so den Analysenablauf wesentlich zu vereinfachen. So kann z.B. bei der Analyse von Serum das Jodat den gebräuchlichen schwach sauren Enteiweissungsmitteln wie z.B. Uranylacetat zugesetzt werden, wodurch man eine weitgehend entstörte Testlösung erhält.

Die diagnostischen Mittel sind vorzugsweise Schnellteste, bei denen das Reagentiensystem in einem saugfähigen, in dem Testsystem unlöslichen Träger imprägniert oder in einem im Testsystem quellbaren Film incorporiert ist, der gegebenenfalls auf einem festen Träger, beispielsweise einer Kunststoff-Folie, befestigt ist. Die Reaktion wird dann nach Befeuchten mit dem Testsystem an der eintretenden Verfärbung bestimmt.

Die diagnostischen Mittel können jedoch auch in dem Testsystem löslich sein und beispielsweise als Lösung, Lyophilisat oder Reagenztablette vorliegen oder in einem im Testsystem löslichen Film incorporiert sein, der sich seinerseits auf oder in einem festen Träger befindet. Die Reagentien werden dann mit dem Testsystem sowie gegebenenfalls weiterem Lösungsmittel vermischt und die Reaktion in einer Küvette photometrisch bestimmt.

Unter Testsystem wird dabei die zu untersuchende Probe gegebenenfalls unter Zusatz geeigneter Lösungsmittel verstanden. Unter Reagentiensystem wird die Summe der mit der Probe reagierenden Stoffe und aller sonstigen Hilfsstoffe wie Puffer, Netzmittel, viskositätsregulierende Stoffe, Stabilisierungsmittel, Kontrastfärbemittel sowie gegebenenfalls Lösungsmittel etc. verstanden.

In den folgenden Beispielen soll die Erfindung näher erläutert werden, ohne sie jedoch auf das Offenbarte einzugrenzen.

Beispiel 1
Testpapier zum Nachweis von Blut (Erythrocyten) im Harn
Filterpapier (Schleicher & Schüll Nr. 23 SL) wird nacheinander mit folgenden Lösungen imprägniert und bei 40°C getrocknet:

Lösung 1

| | |
|---|---|
| 1,2 molarer Citratpuffer vom pH 5,25 | 35,0 ml |
| Ethylendiamintetraessigsäure, Dinatrium-salz | 0,1 g |
| Dioctylnatriumsulfosuccinat | 0,5 g |
| 2,5-Dimethylhexan-2,5-dihydroperoxid (ca. 70%ig) | 1,6 g |
| Phosphorsäuretrimorpholid | 12,7 g |
| Natriumjodat | 0,5 g |
| Ethanol | 30,0 ml |
| dest. Wasser | ad 100,0 ml |

Lösung 2

| | |
|---|---|
| 3,3′,5,5′-Tetramethylbenzidin | 0,3 g |

| Phenanthridin | 0,2 g |
|---|---|
| 1-Phenylsemicarbazid | 0,02 g |
| Toluol/Methanol (60:40) | ad 100,0 ml |

Mit diesem Testpapier kann die Anwesenheit von 5 Ery/mm³ noch in Gegenwart von 150–200 mg Ascorbinsäure/dl nachgewiesen werden. Bei einem analogen Testpapier, das anstelle des Jodats $3 \cdot 10^4$ U Ascorbatoxidase enthält, ist die positive Reaktion noch bis zu einer Konzentration von 30–50 mg Ascorbinsäure/dl, bei einem Papier ohne Zusätze nur bis ca. 10 mg Ascorbinsäure/dl sichtbar. Wird das erfindungsgemässe Testpapier 3 Tage lang auf 60°C erhitzt, so behält es seine Empfindlichkeit bei. Das Papier mit Ascorbatoxidase reagiert nach dieser Belastung nur noch wie das Papier ohne Zusätze.

Beispiel 2
Testpapier zur semiquantitativen Bestimmung von Glucose im Harn
Filterpapier (Schleicher & Schüll 597 NF-Ind.) wird nacheinander mit Lösungen folgender Zusammensetzung getränkt und jeweils bei 50°C getrocknet:

Lösung 1

| Glucoseoxidase (71 U/mg) | 1,2 g |
|---|---|
| Peroxidase (66 U/mg) | 0,2 g |
| 1,2 m Citratpuffer pH 5 | 50,0 ml |
| 9-(γ-Dimethylaminopropyl)-6-chlor-3-aminocarbazol-dihydrochlorid | 2,1 g |
| Tartrazin | 0,12 g |
| Laurolsarkosin | 1,1 g |
| Wasser dest. | ad 100,0 ml |

Lösung 2

| Tetramethylammonium-jodat | 1,4 g |
|---|---|
| Ethanol | ad 100,0 ml |

Auf die gleiche Weise wird ein Testpapier hergestellt, das nur mit Lösung 1 imprägniert ist.

Es werden Urine mit 100, 300 und 1000 mg Glucose/dl hergestellt, in die jeweils 0, 50, 100 und 200 mg Ascorbinsäure/dl eingewogen werden.

Die Testpapiere werden eingetaucht und auf eine saugende Unterlage gelegt. Eine Minute nach dem Eintauchen werden ihre Reaktionsfarben verglichen, wobei die des Urins ohne Ascorbinsäure als innerer Standard gewählt wurde. Aus der folgenden Tabelle ergibt sich, dass die Störung durch Ascorbinsäure durch den Zusatz von Jodat beseitigt wird.

| Anzeige ausgedrückt in mg Glucose/dl | | | |
|---|---|---|---|
| Jodat | 0 | 50 | 100 | 200 mg Ascorbinsäure/dl |
| − | 100 | neg. | neg. | neg. |
| + | 100 | 100 | 100 | 100 |
| − | 300 | 100 | neg. | neg. |
| + | 300 | 300 | 300 | 300 |
| − | 1000 | 300 | 100 | neg. |
| + | 1000 | 1000 | 1000 | 1000 |

Beispiel 3
Testpapier zum Nachweis von Glucose im Harn
Filterpapier (Schleicher & Schüll 597 NF) wird mit einer Lösung folgender Zusammensetzung getränkt und bei 50°C getrocknet:

| Glucoseoxidase (71 U/ml) | 0,38 g |
|---|---|
| Peroxidase (66 U/ml) | 0,02 g |
| Kaliumjodat | 2,00 g |
| Tartrazin | 0,08 g |
| o-Tolidin | 0,42 g |
| Ethanol | 33,0 ml |
| Wasser dest | ad 100,0 ml |

Mit diesem Testpapier geben Urine mit 50 mg Glucose/dl, denen 0, 50, 100 und 200 mg Ascorbinsäure/dl zugewogen wurde, praktisch die gleiche grüne Reaktionsfarbe.
Ein analoges Testpapier ohne Jodat gibt nur im ascorbinsäurefreien Urin eine positive Reaktion.

Beispiel 4
Testfilm zur Bestimmung geringer Glucosegehalte in Blut oder Serum

Bestandteile

| Polyvinylacetatpropionatdispersion (Propiofan 70 D) | 45,0 g |
|---|---|
| 1,85%ige Lösung von Natriumalginat in 0,5 m Phosphatpuffer vom pH 5,5 | 35,0 g |
| Natriumnonylsulfat in 5,0 ml Wasser gelöst | 0,75 g |
| Glucoseoxidase (71 U/mg) in 10 ml Wasser gelöst | 0,2 g |
| Peroxidase (66 U/mg) | 0,25 g |
| 3,3′,5,5′-Tetramethylbenzidin in 5 ml Aceton gelöst | 0,68 g |
| Natriumjodat | 1,0 g |

Die Bestandteile werden gut durchgemischt, in einer Schichtdicke von 200 μ auf einer Unterlage aus Kunststoff-Folie ausgestrichen und 35 Minuten bei 60°C getrocknet.
Auf gleiche Weise wurde ein Film ohne Jodat hergestellt.
Seren mit 20 mg Glucose/dl und 0, 2,5 bzw. 5,0 mg Ascorbinsäure/dl werden aufgetropt, nach 1 Minute wird abgewischt und nach weiteren 2 Minuten wird die Färbung an einem handelsüblichen Remissionsphotometer (Reflomat®) unter Verwendung einer linearen 0–100-Skala gemessen:

| Testfilm Serum | | | |
|---|---|---|---|
| mit | 0 | 2,5 | 5,0 mg Ascorbinsäure/dl |
| ohne Jodat | 47 | 43 | 35 |
| mit Jodat | 46 | 45 | 45 |

**Beispiel 5**

Testpapier zum Nachweis von NADH
Filterpapier (Schleicher & Schüll 23 SL) wird mit einer Lösung folgender Zusammensetzung imprägniert und bei 50°C getrocknet:

| | |
|---|---|
| Jod-nitro-triphenyltetrazoliumchlorid | 0,2 g |
| Natriumjodat | 0,5 g |
| Nonylphenolpolyglykolether | 0,2 g |
| Diaphorase (32 U/mg) | 0,05 g |
| 0,15 m Phosphatpuffer pH 7 | 40,0 ml |
| Wasser dest. | ad 100,0 ml |

In gleicher Weise wurde ein Testpapier ohne Jodat hergestellt.

Mit wässrigen Lösungen von NADH reagieren beide Papiere mit der gleichen roten Färbung. Fügt man den NADH-Lösungen Ascorbinsäure zu, so reagieren die Papiere ohne Jodat stärker.

**Beispiel 6**
Bestimmung von Glucose in Serum

Lösungen

Enteiweissungslösung 1:
0,16% Uranylacetat in 0,9%iger Kochsalzlösung

Enteiweissungslösung 2:
0,16% Uranylacetat und 0,05% Natriumjodat in 0,9%iger Kochsalzlösung

Reagenzlösung:
POD 0,8 U/ml, GOD 10 U/ml, Azino-bis-benzthiazolonsulfonsäure, $NH_4$-Salz 1,0 mg/ml in Phosphatpuffer pH 7 (100 mmol/l)

Probe 1:
Serum mit 100 mg Glucose/dl

Probe 2:
Serum mit 100 mg Glucose/dl und 20 mg Ascorbinsäure/dl

Standard:
9,1 mg Glucose/100 ml Wasser

Enteiweissung
Gemäss folgendem Schema (ml) pipettieren und zentrifugieren:

| | | | | |
|---|---|---|---|---|
| Enteiweissungslösung 1 | 1,00 | 1,00 | – | – |
| Enteiweissungslösung 2 | – | – | 1,00 | 1,00 |
| Probe 1 | 0,10 | – | 0,10 | – |
| Probe 2 | – | 0,10 | – | 0,10 |
| ergibt Überstand Nr. | 1.1 | 1.2 | 2.1 | 2.2 |

Analyse
Gemäss folgendem Schema pipettieren (ml), 30 Minuten bei 25°C inkubieren, Extinktion bei 436 nm messen (d = 1 cm)

| | Leerwert | Überstand Standard | 1.1 | 1.2 | 2.1 | 2.2 |
|---|---|---|---|---|---|---|
| dest. Wasser | 0,1 | – | – | – | – | – |
| Standard | – | 0,1 | – | – | – | – |
| Überstand | – | – | 0,1 | 0,1 | 0,1 | 0,1 |
| Reagenzlösung | 5,0 | 5,0 | 5,0 | 5,0 | 5,0 | 5,0 |
| E | 0,115 | 0,425 | 0,424 | 0,366 | 0,426 | 0,423 |
| E-E (Leerwert) | – | 0,310 | 0,309 | 0,251 | 0,311 | 0,308 |

Ergebnis
Berechnet nach C = 100 · E (Probe)/E (Standard)

| Überstand | 1.1 | 1.2 | 2.1 | 2.2 |
|---|---|---|---|---|
| Ascorbinsäure in Probe | – | + | – | + |
| Jodat in Enteiweissungslösung | – | – | + | + |
| Ergebnis (mg/dl) | 99,7 | 89,0 | 100,3 | 99,4 |

Die durch Ascorbinsäure bewirkte Störung in der Probe 2 wird also durch Jodat völlig aufgehoben.

Beispiel 7
Bestimmung von L-Glutaminsäure

Lösungen

Reagenzlösung 1:
1,2 ml Triton X 100
30 U Diaphorase
10 mg NAD
60 mg Jod-nitro-triphenyltetrazoliumchlorid in 100 ml 0,1 m Kaliumphosphat/Triethanolamin-Puffer pH 8,6

Reagenzlösung 2:
90 000 U Glutamatdehydrogenase in 100 ml Wasser

Probe 1:
100 mg L-Glutaminsäure in 100 ml Wasser

Probe 2:
100 mg L-Glutaminsäure und 40 mg Ascorbinsäure in 100 ml Wasser

Jodat-Lösung:
200 mg Natriumjodat in 100 ml Wasser

Probenvorbereitung
Gemäss folgendem Schema pipettieren (ml) und 15 Minuten bei Raumtemperatur stehen lassen:

| | | | | |
|---|---|---|---|---|
| Probe 1 | 1,0 | 1,0 | – | – |
| Probe 2 | – | – | 1,0 | 1,0 |
| NaJO$_3$-Lsg. | – | 1,0 | – | 1,0 |
| dest. Wasser | 1,0 | – | 1,0 | – |
| ergibt Mischung Nr. | 1.1 | 1.2 | 2.1 | 2.2 |

Analyse
Gemäss folgendem Schema pipettieren (ml), nach 2 Minuten Anfangsextinktion E 1 bei 492 nm (d = 1 cm) messen, mit Reagenzlösung 2 starten, nach 15 Minuten Endextinktion E 2 messen.

| | | | Mischung | | |
|---|---|---|---|---|---|
| | Leerwert | 1.1 | 1.2 | 2.1 | 2.2 |
| Reagenzlösung 1 | 1,0 | 1,0 | 1,0 | 1,0 | 1,0 |
| dest. Wasser | 2,0 | 1,8 | 1,8 | 1,8 | 1,8 |
| Mischung | – | 0,2 | 0,2 | 0,2 | 0,2 |
| E 1 | 0,058 | 0,058 | 0,053 | Schleich | 0,041 |
| Reagenzlösung 2 | 0,03 | 0,03 | 0,03 | 0,03 | 0,03 |
| E 2 | 0,062 | 0,490 | 0,499 | Schleich | 0,503 |
| E2–E1 ($\Delta$E) | 0,004 | 0,432 | 0,446 | – | 0,454 |
| $\Delta$E–$\Delta$E (Leerwert) | – | 0,428 | 0,442 | – | 0,452 |

Ergebnis

| Berechnet nach C = 224.($\Delta$E–$\Delta$E (Leerwert)) | | | | |
|---|---|---|---|---|
| Mischung Nr. | 1.1 | 1.2 | 2.1 | 2.2 |
| Ascorbinsäure in Probe | – | – | + | + |
| Jodat-Behandlung | – | + | – | + |
| Ergebnis (mg/dl) | 95,9 | 99,0 | nicht ablesbar | 101,2 |

Der durch Ascorbinsäure verursachte Schleich der Extinktion (langsame Reduktion des Tetrazoliumsalzes), der eine exakte Messung verhindert, kann durch Vorinkubation der Probenlösung mit Jodat verhindert werden, ohne dass das überschüssige Jodat die Analyse stört.

**Patentansprüche**

1. Verfahren zum Nachweis von Redox-Reaktionen durch Einbringen eines Redox-Reagentiensystems in ein Testsystem, dadurch gekennzeichnet, dass zusätzlich ein im Testsystem lösliches Jodat in einer Menge zugegeben wird, die einem Überschuss, bezogen auf die höchste im Testsystem vorkommende Menge von störender Ascorbinsäure entspricht und die Gesamtmischung einen pH-Wert von 5–9 aufweist.

2. Verfahren nach Anspruch 1, dadurch gekennzeichnet, dass der pH-Wert des Testsystems durch die Zugabe des Redox-Reagentiensystems erhöht wird.

3. Verfahren nach Anspruch 2, dadurch gekennzeichnet, dass der pH-Wert von 5–6 auf 7–9 erhöht wird.

4. Diagnostisches Mittel zum Nachweis von Redox-Reaktionen, enthaltend ein Redox-Reagentiensystem, dadurch gekennzeichnet, dass zusätzlich ein im Testsystem lösliches Jodat in einer Menge enthalten ist, die einem Überschuss, bezogen auf die höchste im Testsystem vorkommende Menge von störender Ascorbinsäure entspricht und das Reagentiensystem einen Puffer enthält, der zusammen mit dem Testsystem einen pH-Wert von 5–9 einstellt.

5. Diagnostisches Mittel nach Anspruch 4, dadurch gekennzeichnet, dass 0,5–2 g Jodat pro 100 ml Testsystem enthalten ist.

6. Diagnostisches Mittel nach einem der Ansprüche 4–5, dadurch gekennzeichnet, dass das Redox-Reagentiensystem aus einem Oxidationsindikator, einem Hydroperoxid, einer Peroxidase und üblichen Hilfsstoffen besteht.

7. Diagnostisches Mittel nach einem der Ansprüche 4–5, dadurch gekennzeichnet, dass das Redox-Reagentiensystem aus einem Reduktionsindikator, einem Reduktionsmittel sowie gegebenenfalls einem Elektronenüberträger besteht.

## Claims

1. Process for the detection of redox reactions by the introduction of a redox reagent system into a test system, characterised in that an iodate soluble in the test system is additionally introduced in an amount which corresponds to an excess, referred to the highest amount of disturbing ascorbic acid present in the test system, and the total mixture has a pH value of 5–9.

2. Process according to claim 1, characterised in that the pH value of the test system is increased by the addition of the redox reagent system.

3. Process according to claim 2, characterised in that the pH value is increased from 5–6 to 7–9.

4. Diagnostic agent for the detection of redox reactions containing a redox reagent system, characterised in that an iodate soluble in the test system is additonally present in an amount which corresponds to an excess, referred to the highest amount of disturbing ascorbic acid present in the test system, and the reagent system contains a buffer which, together with the test system, gives a pH value of 5–9.

5. Diagnostic agent according to claim 4, characterised in that 0.5–2 g of iodate is present per 100 ml of test system.

6. Diagnostic test system according to one of claims 4–5, characterised in that the redox reagent system consists of an oxidation indicator, a hydroperoxide, a peroxidase and conventional adjuvants.

7. Diagnostic agent according to one of claims 4–5, characterised in that the redox reagent system consists of a reduction indicator, a reducing agent, as well as possibly an electron carrier.

## Revendications

1. Procédé pour la mise en œuvre de réactions redox en introduisant un système de réactifs redox dans un système de test, caractérisé en ce qu'on ajoute additionnellement un iodate soluble dans le système de test en une quantité correspondant à la quantité maximale d'acide ascorbique perturbateur présente dans le système de test, et en ce que le pH du mélange entier est compris entre 5 et 9.

2. Procédé selon la revendication 1, caractérisé en ce que le pH du système de test est augmenté par l'addition d'un système de réactifs redox.

3. Procédé selon la revendication 2, caractérisé en ce que le pH compris entre 5 et 6 est porté à une valeur comprise entre 7 et 9.

4. Agent de diagnostic pour la mise en évidence de réactions redox comportant un système de réactifs redox, caractérisé en ce que l'agent comporte additionnellement un iodate soluble dans le système de test en une quantité correspondant à un excès par rapport à la quantité maximale d'acide ascorbique perturbateur présente dans le système de test, et en ce que le système de réactifs contient un tampon ajustant en coopération avec le système de test, le pH entre 5 et 9.

5. Agent de diagnostic selon la revendication 4, caractérisé en ce qu'il comporte entre 0,5 et 2 g d'iodate pour 100 ml de système de test.

6. Agent de diagnostic selon l'une des revendications 4 et 5, caractérisé en ce que le système de réactifs redox est composé d'un indicateur d'oxydation, d'un hydroperoxyde, d'une peroxydase et des agents auxiliaires habituels.

7. Agent de diagnostic selon l'une des revendications 4 et 5, caractérisé en ce que le système de réactifs redox est composé d'un indicateur de réduction, d'un agent réducteur ainsi qu'éventuellement d'un agent de transfert d'électrons.